# EUROPEAN PATENT APPLICATION

(11) **EP 3 410 100 A1**
(43) Date of publication of application: **05.12.2018**
(21) Application number: 16888019.3
(22) Date of filing: 21.06.2016
(51) Int. Cl.: G01N 21/65, G01N 33/15

(54) **ANALYSIS METHOD AND ANALYZING DEVICE**

(30) Priority: 27.01.2016 JP 2016013679
(71) Applicant: HORIBA, LTD., Kyoto-shi, Kyoto 601-8510 (JP)
(72) Inventor: TSUMOTO, Kouhei, Tokyo 113-0024 (JP); OTA, Chikashi, Kyoto-shi Kyoto 601-8510 (JP); NOGUCHI, Shintaro, Kyoto-shi Kyoto 601-8510 (JP)
(74) Representative: Isarpatent
(86) International application number: PCT/JP2016/068363
(87) International publication number: WO 2017/130433

(57) **Abstract**

To provide an analysis method and an analysis device for analyzing a protein solution utilizing Raman spectroscopy in order to evaluate whether the protein solution is suitable for medicine or not.

Raman spectra of protein solutions having a plurality of concentrations are acquired. An index, which changes according to interaction between protein molecules, is calculated based on a predetermined peak included in the Raman spectra. A threshold of concentration at which protein can be denatured is specified based on change in the index against change in concentration. The threshold is compared with a concentration of the protein solution required as medicine such as antibody drug, and whether the protein solution is suitable as medicine or not is determined. When the concentration required as medicine is equal to or higher than the threshold, for example, it is judged that the protein solution is not suitable as medicine. When the protein solution is not suitable as medicine, analysis can be repeated by changing a condition of the protein solution.

## Description

### [Technical Field]

The present invention relates to a method of analyzing a high-concentration protein solution utilizing Raman spectroscopy, and an analysis device.

### [Background Art]

In recent years, medicines using biological material such as nucleic acid or protein have been developed. For example, there is an antibody drug which uses an antibody. An antibody is a type of protein, and a medicine, such as an antibody drug, which uses protein is in the form of a protein solution. A protein solution to be used as medicine is produced as a high-concentration solution in order to exhibit an effect in the body of a patient. In a case where the concentration of a protein solution becomes high, protein denaturation, such as protein structural change or protein aggregation due to interaction between protein molecules, sometimes occurs. Since an effect as medicine changes when protein is denatured, it is required to measure the state of a high-concentration protein solution, such as whether the protein structure has changed or not, for the purpose of manufacture and quality control of antibody drug.

Since it is difficult to measure the state of a high-concentration protein solution, it has been a conventional method to presume the state of a high-concentration protein solution by measuring a diluted protein solution and extrapolating the measurement result. However, this method cannot achieve accurate measurement of the state of a high-concentration protein solution. Methods of directly measuring the state of a high-concentration protein solution include a small angle X-ray scattering method and a neutron scattering method. Moreover, Patent Literature 1 discloses use of Raman spectroscopy for antibody purification.

### [Prior Art Document]

### [Patent Document]

[Patent Document 1] Japanese Unexamined Patent Application Publication No. 2013-537235

### [Summary of Invention]

### [Problems to be Solved by Invention]

The small angle X-ray scattering method and the neutron scattering method have a problem that a large-scale device is used and this increases the measurement cost, and a problem that information on the structure of protein cannot be obtained. In contrast, it is possible with Raman spectroscopy to make the measurement cost relatively low and to obtain information on the structure of protein by directly measuring the state of a high-concentration protein solution. However, a technique for analyzing the state of a high-concentration protein solution using Raman spectroscopy and evaluating whether a protein solution is suitable for medicine or not has not been established yet.

The present invention has been made with the aim of solving the above problems, and an object thereof is to provide an analysis method and an analysis device for analyzing a protein solution utilizing Raman spectroscopy in order to evaluate whether the protein solution is suitable for medicine or not.

### [Means for Solving Problems]

An analysis method according to the present invention for analyzing a protein solution utilizing Raman spectroscopy is characterized by comprising the steps of: acquiring a Raman spectrum of each of protein solutions, which contain a specific protein dissolved therein and have a plurality of concentrations, by Raman spectroscopy; calculating an index, which increases and decreases according to interaction between protein molecules in the protein solution, based on one or a plurality of predetermined peaks included in the Raman spectrum for each concentration; specifying a concentration range, in which a variation of the index against increase in concentration falls in a predetermined range, based on the index calculated regarding each concentration; specifying a threshold of concentration, which is to be used as a criterion for determining whether the specific protein is denatured or not, based on the concentration range; and comparing the threshold with a specific concentration value set for the specific protein.

An analysis method according to the present invention for analyzing a protein solution utilizing Raman spectroscopy is characterized by comprising the steps of: acquiring a Raman spectrum of each of protein solutions, which contain a specific protein dissolved therein and have a plurality of concentrations, by Raman spectroscopy; calculating an index, which increases and decreases according to interaction between protein molecules in the protein solution, based on one or a plurality of predetermined peaks included in the Raman spectrum for each concentration; and specifying a threshold of concentration, which is to be used as a criterion for determining whether the specific protein is denatured or not, based on change in the index against change in concentration.

In the analysis method according to the present invention, the index is an intensity ratio of two predetermined peaks in the Raman spectrum, the intensity ratio indicating a degree to which an OH group of tyrosine in protein acts as a hydrogen acceptor while acting also as a hydrogen donner.

In the analysis method according to the present invention, the index is an intensity ratio of two predetermined peaks in the Raman spectrum, the intensity ratio indicating a degree to which an N-H site of an indole ring included in tryptophan in protein acts as a hydrogen acceptor while acting also as a hydrogen donner.

In the analysis method according to the present invention, the index is a width of a predetermined peak in the Raman spectrum, the width indicating a width of a predetermined structural angle of tryptophan in protein.

In the analysis method according to the present invention, the index is an intensity ratio of a predetermined peak resulting from phenylalanine in protein and a predetermined peak resulting from an amide group.

The analysis method according to the present invention is characterized by further comprising the steps of: comparing a peak shape of a predetermined peak resulting from an amide group in protein between Raman spectra regarding a plurality of concentrations; determining whether the peak shape at a high concentration changes more in comparison with the peak shape at a low concentration or not; and judging that the protein solution is not suitable for medicine at a concentration equal to or higher than a concentration at which the peak shape changes in a case where the peak shape at the high concentration changes more in comparison with the peak shape at the low concentration.

The analysis method according to the present invention is characterized by further comprising the steps of: determining whether the protein solution is suitable for medicine or not based on a result of comparison between the threshold and the specific concentration value; and repeating analysis of the protein solution, which is prepared by adjusting a component other than the specific protein, in a case where the protein solution is not suitable for medicine.

An analysis device according to the present invention for analyzing a protein solution based on a Raman spectrum is characterized by comprising: a storage unit configured to store a specific concentration value set for a specific protein, and a Raman spectrum acquired for each of protein solutions, which contain the specific protein dissolved therein and have a plurality of concentrations; a calculation unit configured to calculate an index, which increases and decreases according to interaction between protein molecules in the protein solution, based on one or a plurality of predetermined peaks included in the Raman spectrum for each concentration; a first specification unit configured to specify a concentration range, in which a variation of the index against increase in concentration falls in a predetermined range, based on the index calculated regarding each concentration; a second specification unit configured to specify a threshold of concentration, which is to be used as a criterion for determining whether the specific protein is denatured or not, based on the concentration range; and a comparison unit configured to compare the threshold with the specific concentration value.

An analysis device according to the present invention for analyzing a protein solution based on a Raman spectrum is characterized by comprising: a storage unit configured to store a specific concentration value set for a specific protein, and a Raman spectrum acquired for each of protein solutions, which contain the specific protein dissolved therein and have a plurality of concentrations; a calculation unit configured to calculate an index, which increases and decreases according to interaction between protein molecules in the protein solution, based on one or a plurality of predetermined peaks included in the Raman spectrum for each concentration; and a specification unit configured to specify a threshold of concentration, which is to be used as a criterion for determining whether the specific protein is denatured or not, based on change in the index against change in concentration.

An analysis device according to the present invention for analyzing a protein solution is characterized by comprising: a storage unit configured to store a threshold of concentration, which is preliminarily specified utilizing Raman spectroscopy for a protein solution containing a specific protein dissolved therein and is to be used as a criterion for determining whether the specific protein is denatured or not; and a comparison unit configured to compare a specific concentration value, which is set for the specific protein, with the threshold.

In the present invention, Raman spectra of protein solutions having a plurality of concentrations are acquired, a concentration range, in which an index corresponding to interaction between protein molecules does not substantially change against change in concentration, is specified on the basis of the Raman spectra, and a threshold of concentration, which is to be used as a criterion for determining whether protein is denatured or not, is specified and compared with a concentration of a protein solution required as medicine. Within the concentration range in which the index does not substantially change against concentration, interaction between protein molecules may possibly be saturated and this may possibly cause denaturation of protein, and it is possible to specify a threshold of concentration, at which protein can be denatured, on the basis of the concentration range. By comparing the specified threshold with a concentration of a protein solution required as medicine, it is possible to evaluate the state of a protein solution at a concentration required as medicine.

In the present invention, Raman spectra of protein solutions having a plurality of concentrations are acquired, an index corresponding to interaction between protein molecules is calculated on the basis of the Raman spectra, and a threshold of concentration, which is to be used as a criterion for determining whether protein is denatured or not, is specified on the basis of change in the index in response to change in concentration. It is possible to specify a threshold of concentration, at which protein can be denatured, on the basis of change in an index against change in concentration, the index is obtained from the Raman spectra, such as an index, which becomes substantially constant when interaction between protein molecules is saturated.

Moreover, in the present invention, an index, which indicates the degree to which an OH group of tyrosine acts as a hydrogen acceptor while acting also as a hydrogen donner, is used. In a case where protein molecules interact with each other, an OH group of tyrosine acts both as a hydrogen donner and as a hydrogen acceptor. In a case where this index does not substantially change against change in concentration, interaction between protein molecules is saturated. Therefore, by using this index, it is possible to specify a concentration range in which interaction between protein molecules is saturated.

Moreover, in the present invention, an index, which indicates the degree to which an N-H site of an indole ring included in tryptophan acts as a hydrogen acceptor while acting also as a hydrogen donner, is used. In a case where protein molecules interact with each other, the N-H site acts both as a hydrogen donner and as a hydrogen acceptor. In a case where this index does not substantially change against change in concentration, interaction between protein molecules is saturated. Therefore, by using this index, it is possible to specify a concentration range in which interaction between protein molecules is saturated.

Moreover, in the present invention, an index, which indicates a width of a predetermined structural angle of tryptophan, is used. When interaction between protein molecules is strong, the interaction causes change in the angle, increase in dispersion of the angle, and widening of a distribution width of the angle. In a case where this index does not substantially change against change in concentration, interaction between protein molecules is saturated. Therefore, by using this index, it is possible to specify a concentration range in which interaction between protein molecules is saturated.

Moreover, in the present invention, an intensity ratio between a predetermined peak resulting from phenylalanine in protein and a predetermined peak resulting from an amide group is used as an index. This index is substantially constant at a relatively low concentration and changes at a concentration at which protein aggregates. In a case where this index changes against change in concentration, protein aggregates. Therefore, by using this index, it is possible to specify a concentration range in which protein aggregates.

Moreover, in the present invention, it is judged that a protein solution having a concentration equal to or higher than a concentration at which a peak shape changes is not suitable for medicine in a case where the peak shape of a peak resulting from an amide group changes at a high concentration. Change of the peak shape indicates that the entire structure of protein is not maintained in the protein solution. Since the entire structure of protein is not maintained and an effect exhibited by protein changes at a concentration equal to or higher than a concentration at which the peak shape changes, it is judged that the protein solution is not suitable as medicine at a concentration required as medicine.

Moreover, in the present invention, whether a protein solution is suitable for medicine or not is determined on the basis of a result of comparison between a specified threshold and a concentration of the protein solution required as medicine. In a case where it is judged that the protein solution is not suitable for medicine, analysis is repeated for a protein solution obtained by adjusting a component other than protein. By adjusting a component other than protein, it is possible to change a condition, such as pH, of the protein solution. When a condition of a protein solution is changed, a concentration at which protein is denatured sometimes changes. By making analysis every time a condition of a protein solution is changed, it is possible to find a condition of the protein solution suitable as medicine.

### [Advantageous Effect of Invention]

With the present invention, it is possible to evaluate whether a specific protein solution is suitable for medicine or not by, for example, judging that the protein solution is not suitable for medicine in a case where a concentration required as medicine is equal to or higher than a threshold of concentration at which protein can be denatured. Accordingly, the present invention exhibits excellent effects such as an effect that it becomes possible to easily evaluate whether a protein solution containing protein such as an antibody dissolved therein is suitable for medicine or not.

### [Brief Description of Drawings]

FIG. 1 is a conceptual diagram illustrating procedures of an analysis method of a protein solution according to Embodiment 1;
FIG. 2 is a block diagram illustrating the structure of a Raman spectroscopic device utilized in Embodiment 1;
FIG. 3 is a characteristic diagram illustrating an example of Raman spectra;
FIG. 4 is a block diagram illustrating the inner structure of an analysis device according to Embodiment 1;
FIG. 5 is a characteristic diagram in which a Raman spectrum of a protein solution is enlarged;
FIG. 6 is a view illustrating the chemical structural formula of tryptophan;
FIG. 7 is a flowchart illustrating procedures of processing to be executed by an analysis device according to Embodiment 1;
FIG. 8 is a characteristic diagram illustrating an example (black circles) of change in intensity ratio (856/837 cm⁻¹) of peaks at 856 cm⁻¹ and 837 cm⁻¹ included in the Raman band of tyrosine against concentration, and an example (white circles) of change in intensity ratio (870/877 cm⁻¹) of peaks at 870 cm⁻¹ and 877 cm⁻¹ included in the Raman band of tryptophan against concentration;
FIG. 9 is a characteristic diagram illustrating an example of change in peak width at 1555 cm⁻¹ included in the Raman band of tryptophan against concentration;
FIG. 10 is a block diagram illustrating the inner structure of an analysis device according to Embodiment 2;
FIG. 11 is a flowchart illustrating procedures of processing to be executed by an analysis device according to Embodiment 2;
FIG. 12 is a flowchart illustrating procedures of processing to be executed by an analysis device according to Embodiment 3;
FIG. 13 is a characteristic diagram illustrating an example of Raman spectra of amide I of aqueous solutions of IgG;
FIG. 14 is a characteristic diagram illustrating an example of change in intensity ratio of 856/830 cm⁻¹ obtained from Raman spectra of tyrosine in aqueous solutions of IgG against concentration;
FIG. 15 is a characteristic diagram illustrating an example of change in peak width at 1550 cm⁻¹ obtained from Raman spectra of tryptophan in aqueous solutions of IgG against concentration; and
FIG. 16 is a characteristic diagram illustrating an example of change in intensity ratio of 1004/1240 cm⁻¹ obtained from Raman spectra of an aqueous solution of IgG against concentration.

### [Mode for Carrying out Invention]

The following description will concretely explain the present invention with reference to the drawings illustrating some embodiments thereof.

### (Embodiment 1)

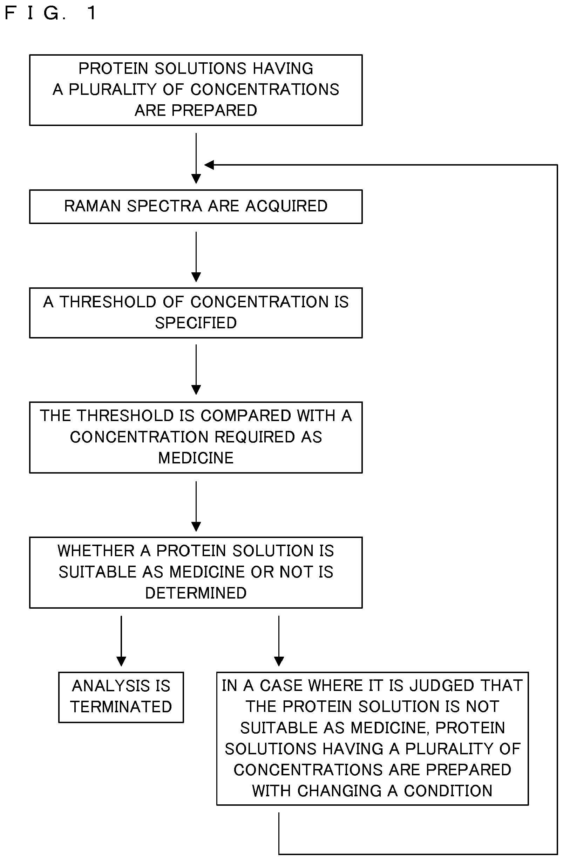

A protein solution to be used as medicine such as antibody drug have a given concentration required to exhibit an effect in the body of a patient. In the present invention, a threshold of concentration at which protein in a protein solution can be denatured is specified utilizing Raman spectroscopy, and whether the protein solution is suitable for medicine or not is evaluated by comparing the specified threshold with the concentration required as medicine. Protein to be analyzed in the present invention includes peptide. In a case where a protein solution has a high concentration above a certain degree, interaction between protein molecules sometimes causes denaturation of protein, such as change of the structure of protein or aggregation of protein. A threshold of concentration at which protein can be denatured is a threshold of concentration to be used as a criterion for determining whether protein is denatured at each concentration or not. In a case where the concentration of a protein solution is equal to or higher than this threshold, it can be presumed that protein in the protein solution is denatured. FIG. 1 is a conceptual diagram illustrating procedures of an analysis method of a protein solution according to Embodiment 1. An analyzer who analyzes a protein solution first prepares protein solutions, which contain a specific protein dissolved therein and have a plurality of concentrations. A protein solution is an aqueous solution and may include a component other than the specific protein. For example, a protein solution is obtained by dissolving protein in a buffer solution. It is to be noted that an analyzer may not prepare protein solutions but analyze protein solutions prepared by others.

Next, the analyzer acquires Raman spectra of protein solutions having respective concentrations by Raman spectroscopy. For acquiring Raman spectra, a Raman spectroscopic device, which will be described later, is utilized. Next, a threshold of concentration at which protein can be denatured is specified on the basis of the Raman spectra of the respective concentrations. A peak, which changes according to interaction between protein molecules, among a number of peaks included in the Raman spectra does not substantially change against increase in concentration when the concentration increases to a certain degree and interaction is saturated. In a state where interaction between protein molecules is saturated, there is a high possibility that denaturation of protein is caused by interaction. Therefore, by comparing Raman spectra of respective concentrations, it is possible to specify a threshold of concentration at which protein can be denatured. For example, the minimum value of concentration included in a concentration range, in which a Raman spectrum stops changing regardless of increase in concentration, is regarded as a threshold of concentration at which protein can be denatured. Next, the threshold of concentration at which protein can be denatured is compared with a concentration of a protein solution required as medicine. For specification of a threshold and comparison, an analysis device, which will be described later, can be used. Next, whether a protein solution to be analyzed is suitable as medicine or not is determined on the basis of the comparison result. For example, it is judged that protein is denatured at a concentration required as medicine in a case where a concentration required as medicine is equal to or higher than a threshold of concentration at which protein can be denatured. Since an effect as medicine changes in a protein solution in which protein is denatured, the analyzer judges that a protein solution to be analyzed is not suitable as medicine. In contrast, in a case where a concentration required as medicine is lower than a threshold of concentration at which protein can be denatured, it is judged that protein is not denatured at a concentration required as medicine. Since an effect as medicine does not change, the analyzer judges that the protein solution to be analyzed is suitable as medicine.

Analysis is terminated after whether a protein solution is suitable as medicine or not is determined. It is to be noted that the analysis can be continued. In a case where it is judged that the protein solution is not suitable as medicine, the analyzer prepares protein solutions having a plurality of concentrations with changing a condition and repeats the procedures from acquisition of Raman spectra to analysis again. In order to change a condition of a protein solution, a component other than a specific protein in a protein solution is adjusted. For example, a protein solution includes various kinds of acids, alkalis, or salts to be used for adjusting pH, or saccharides as stabilizer in addition to the specific protein, and the amount or type of these components is changed. By changing a condition, a concentration at which protein in a protein solution is denatured sometimes changes. Accordingly, it is possible to find a condition of a protein solution suitable as medicine by making an analysis every time a condition of a protein solution is changed. For example, an analyzer finds such a condition of a protein solution that a threshold of concentration at which protein can be denatured exceeds a concentration required as medicine.

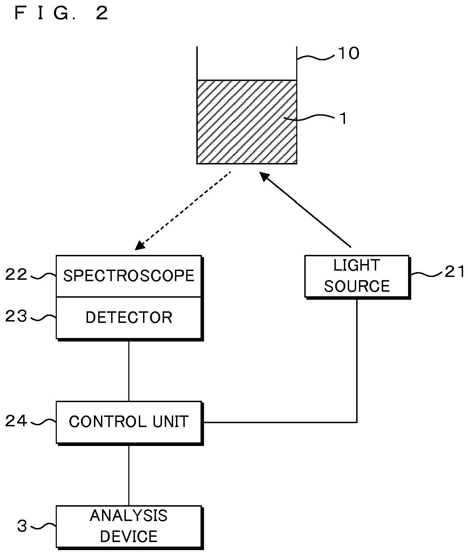

FIG. 2 is a block diagram illustrating the structure of a Raman spectroscopic device utilized in Embodiment 1. A Raman spectroscopic device irradiates a protein solution with monochromatic light and detects Raman scattering light to be generated. The Raman spectroscopic device is provided with a light source 21 configured to emit monochromatic light. The light source 21 is, for example, a laser light source. The light source 21 irradiates a protein solution 1, which is held in a sample cell 10, with monochromatic light. In FIG. 2, monochromatic light emitted from the light source 21 is described with a solid arrow. The Raman spectroscopic device is provided with a spectroscope 22 and a detector 23. Raman scattering light is generated in the protein solution 1 irradiated with monochromatic light, and the generated Raman scattering light enters the spectroscope 22. In FIG. 2, Raman scattering light is described with a dotted arrow. The spectroscope 22 disperses Raman scattering light which has entered the spectroscope 22. The detector 23 detects light having each wavelength dispersed by the spectroscope 22 and outputs a signal corresponding to detection intensity of light having each wavelength. For example, the detector 23 is constituted of a CCD (Charge Coupled Device) optical sensor or a photomultiplier.

Moreover, the Raman spectroscopic device is provided with a control unit 24 configured to control operations of the respective units. The light source 21, the spectroscope 22, and the detector 23 are connected with the control unit 24. On/off of the light source 21 is controlled by the control unit 24. The wavelength of light, which is dispersed by the spectroscope 22 and detected by the detector 23, is controlled by the control unit 24. The detector 23 outputs a signal, which corresponds to the detection intensity of light, to the control unit 24. The control unit 24 receives a signal outputted from the detector 23 and generates a Raman spectrum on the basis of the wavelength of light, which is dispersed by the spectroscope 22, and the detection intensity of light, which is indicated by the inputted signal. An analyzer preliminarily prepares sample cells 10 configured to hold protein solutions 1 having a plurality of concentrations and acquires Raman spectra of the protein solutions 1 having a plurality of concentrations by generating a Raman spectrum with the Raman spectroscopic device every time a sample cell 10 holding a protein solution having each concentration is exchanged.

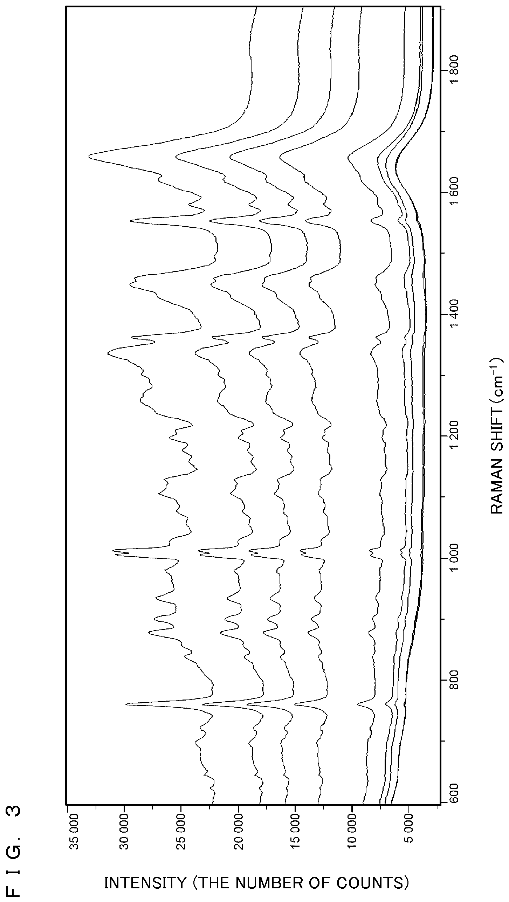

FIG. 3 is a characteristic diagram illustrating an example of Raman spectra. The horizontal axis in the figure represents Raman shift using a unit of wavenumber (cm⁻¹), while the vertical axis represents intensity of Raman scattering light using a unit of the number of counts. A lysozyme solution (protein solution) was prepared by using lysozyme, which was extracted from chicken egg albumen, as an example of protein and dissolving the lysozyme in a tris-hydrochloric acid buffer solution having pH 8.0. The Raman spectra illustrated in FIG. 3 are Raman spectra of lysozyme solutions having concentrations of 2.5, 5, 10, 20, 50, 100, 150, 200, and 300 (mg/ml) in this order from below. In FIG. 3, Raman spectra of concentrations of 2.5 and 5 (mg/ml) are substantially superimposed on each other.

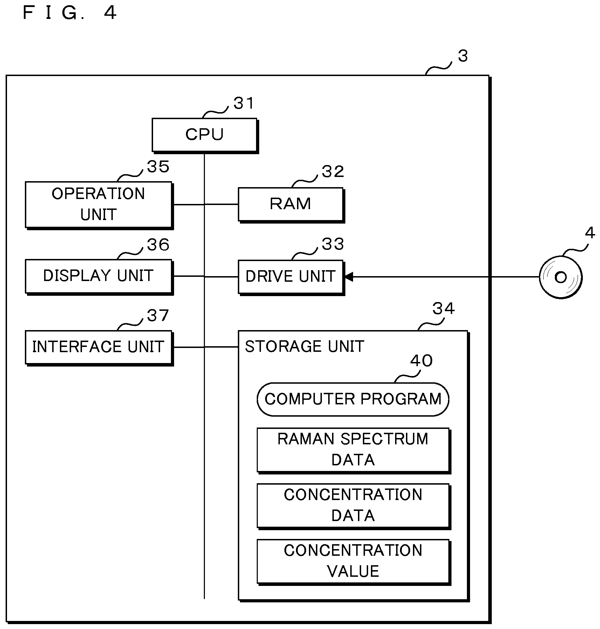

The Raman spectroscopic device is further provided with an analysis device 3 configured to analyze a protein solution. The control unit 24 is connected with the analysis device 3. FIG. 4 is a block diagram illustrating the inner structure of the analysis device 3 according to Embodiment 1. The analysis device 3 is constituted of a computer such as a personal computer. The analysis device 3 is provided with a CPU (Central Processing Unit) 31 configured to perform operations, a RAM (Random Access Memory) 32 configured to store temporal data to be generated in the operations, a drive unit 33 configured to read information from a recording medium 4 such as an optical disk, and a nonvolatile storage unit 34 such as a hard disk. The analysis device 3 is also provided with an operation unit 35 such as a keyboard or a mouse configured to accept an operation by a user, a display unit 36 such as a liquid crystal display, and an interface unit 37. The interface unit 37 is connected with the control unit 24. The CPU 31 causes the drive unit 33 to read a computer program 40 recorded in the recording medium 4 and causes the storage unit 34 to store the read computer program 40. The CPU 31 loads the computer program 40 from the storage unit 34 to the RAM 32 as needed and executes processing necessary for the analysis device 3 according to the loaded computer program 40. It is to be noted that the computer program 40 may be downloaded from outside of the analysis device 3.

The control unit 24 outputs Raman spectrum data, which represents a generated Raman spectrum, to the analysis device 3. The analysis device 3 accepts the Raman spectrum data at the interface unit 37, and the CPU 31 causes the storage unit 34 to store the Raman spectrum data. The storage unit 34 stores Raman spectrum data for each concentration. An analyzer operates the operation unit 35 to input concentration data, which indicates the concentration of a protein solution 1, and the CPU 31 causes the storage unit 34 to store the concentration data. The Raman spectrum data, which represents a Raman spectrum of each concentration, and each concentration indicated by the concentration data are associated with each other. A Raman spectrum and a concentration are associated with each other when an analyzer operates the operation unit 35. Moreover, the storage unit 34 stores a specific concentration value, which indicates a concentration of a protein solution required as medicine. The concentration value is inputted when an analyzer operates the operation unit 35.

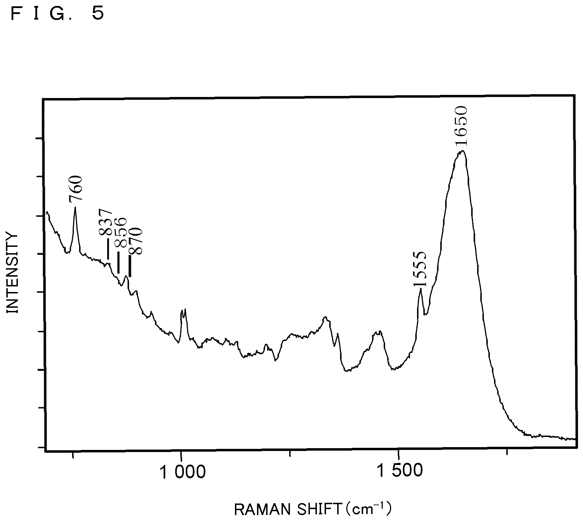

The analysis device 3 performs processing for analyzing a protein solution on the basis of change of a specific peak, which is included in each Raman spectrum, against increase in concentration. FIG. 5 is a characteristic diagram in which a Raman spectrum of a protein solution is enlarged. The horizontal axis in the figure represents Raman shift, while the vertical axis represents intensity of Raman scattering light. A peak at 1650 cm⁻¹ among peaks included in the Raman spectrum is a peak resulting from an amide group in protein. An amide group constitutes the main chain of amino acid in protein. This peak appears in a case where the entire structure of protein is maintained.

Peaks at 856 cm⁻¹ and 837 cm⁻¹ among peaks included in the Raman spectrum result from tyrosine. Tyrosine is one of amino acids, which constitute protein. An intensity ratio (which will be hereinafter described as 856/837 cm⁻¹), which is obtained by dividing the peak intensity at 856 cm⁻¹ by the peak intensity at 837 cm⁻¹, indicates the hydration state of tyrosine. In a case where the intensity ratio of 856/837 cm⁻¹ is as small as a certain degree, this indicates that an OH group included in tyrosine acts as a hydrogen donner. As the intensity ratio becomes large, this indicates that an OH group acts both as a hydrogen donner and as an acceptor. Here, O is carbon, and H is hydrogen. As the concentration of a protein solution becomes high, distance between protein molecules becomes small, and therefore protein molecules interact with each other, an OH group of tyrosine starts acting as an acceptor, and the intensity ratio of 856/837 cm⁻¹ becomes large. That is, the intensity ratio of 856/837 cm⁻¹ indicates the ratio of protein molecules interacting with other protein molecules. At a high concentration above a certain degree, interaction between protein molecules is saturated, and the intensity ratio of 856/837 cm⁻¹ is substantially constant.

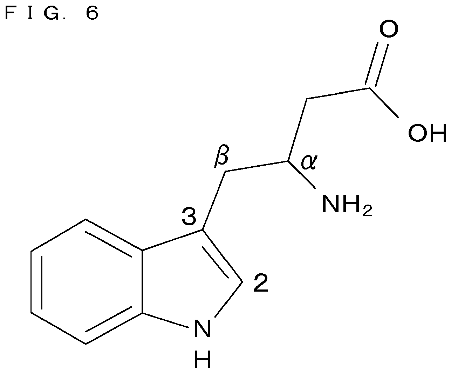

A peak at 1555 cm⁻¹ among peaks included in the Raman spectrum results from tryptophan. Tryptophan is one of amino acids, which constitute protein. More specifically, a peak at 1555 cm⁻¹ results from C=C stretching vibration of an indole ring in tryptophan. Here, C is carbon. FIG. 6 is a figure illustrating the chemical structural formula of tryptophan. A peak at 1555 cm⁻¹ results from C=C stretching vibration between C₂, which is denoted by 2 in FIG. 6, and C₃, which is denoted by 3. The peak width at 1555 cm⁻¹ indicates information on the structure of C₂=C₃-C_{α}-C_{β} including C_{α}, which is denoted by α in FIG. 6, and Cp, which is denoted by β. More specifically, the peak width indicates the state of a torsion angle between C₂=C₃ and C_{α}-C_{β}. In a case where the peak width is small, this indicates that dispersion of a torsion angle is small. In a case where the peak width is large, this indicates that dispersion of a torsion angle is large. As the concentration of a protein solution becomes high, interaction between protein molecules causes change in the torsion angle, increase in dispersion of the torsion angle, widening of a distribution width of the torsion angle, and widening of the peak width of a peak at 1555 cm⁻¹. That is, the peak width at 1555 cm⁻¹ also indicates the ratio of protein molecules interacting with other protein molecules. At a high concentration above a certain degree, interaction between protein molecules is saturated, and the peak width is substantially constant.

Among peaks included in the Raman spectrum, a peak at 877 cm⁻¹ results from an N-H site of an indole ring in tryptophan. Here, N is nitrogen. An N-H site of an indole ring is illustrated in FIG. 6. A peak at 877 cm⁻¹ indicates the state of an N-H site of an indole ring, and the peak width widens to the lower wavenumber side when strong hydrogen bond is made. An intensity ratio (which will be hereinafter described as 870/877 cm⁻¹) obtained by dividing the peak intensity at 870 cm⁻¹ by the peak intensity at 877 cm⁻¹ indicates the state of hydrogen bond in tryptophan. In a case where the intensity ratio of 870/877 cm⁻¹ is as small as a certain degree, this indicates that an N-H site acts as a hydrogen donner. As the intensity ratio becomes large, this indicates that an N-H site acts both as a hydrogen donner and as an acceptor. In a case where a protein solution has a low concentration, an N-H site is mainly surrounded by water and acts as a hydrogen donner by making hydrogen bond with water. In a case where a protein solution has a high concentration, another protein molecule tends to be located around an N-H site, and hydrogen bond between a protein molecule and water is replaced by interaction between protein molecules, hydrogen bond becomes stronger, and an N-H site acts both as a hydrogen donner and as an acceptor. That is, the intensity ratio of 870/877 cm⁻¹ also indicates the ratio of protein molecules interacting with other protein molecules. At a high concentration above a certain degree, interaction between protein molecules is saturated, and the intensity ratio of 870/877 cm⁻¹ is substantially constant.

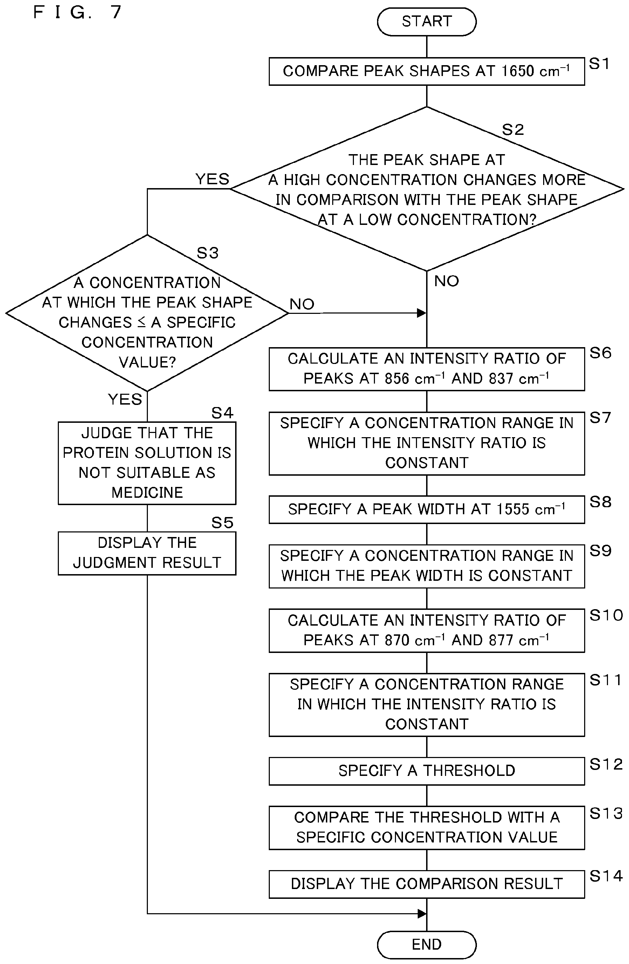

FIG. 7 is a flowchart illustrating procedures of processing to be executed by the analysis device 3 according to Embodiment 1. The CPU 31 executes the following processing according to the computer program 40. The CPU 31 reads out Raman spectrum data of a plurality of concentrations from the storage unit 34 to the RAM 32 and compares peak shapes at 1650 cm⁻¹ included in Raman spectra of a plurality of concentrations with each other (S1). Next, the CPU 31 determines whether the peak shape at a high concentration changes more in comparison with the peak shape at a low concentration or not on the basis of the comparison result (S2). In S1, for example, the CPU 31 normalizes Raman scattering light intensity within a predetermined wavenumber range including 1650 cm⁻¹ for Raman spectra of a plurality of concentrations in a manner such that the peak intensities at 1650 cm⁻¹ become equal to each other, and calculates a difference square sum by squaring and summing up differences of Raman scattering light intensities at each wavenumber within the predetermined wavenumber range between Raman spectra of a plurality of concentrations. The CPU 31 calculates a difference square sum between the minimum concentration and another concentration. In S2, the CPU 31 compares the difference square sum calculated in S1 with a predetermined allowable value, and judges that the peak shape has changed in a case where the difference square sum exceeds the allowable value. The fact that the peak shape at 1650 cm⁻¹ changes more at a high concentration in comparison with a low concentration indicates that the entire structure of protein is not maintained in a high-concentration protein solution. It is presumed that interaction between protein molecules makes it impossible to maintain the entire structure of protein at a high concentration. In a case where the entire structure of protein cannot be maintained, an effect to be exerted by protein changes. In a lysozyme solution, no change was observed in the peak shape at 1650 cm⁻¹.

In a case where the peak shape at 1650 cm⁻¹ has changed (S2: YES), the CPU 31 determines whether a concentration at which the peak shape at 1650 cm⁻¹ changes more in comparison with a case of a low concentration is equal to or lower than a specific concentration value, which indicates a concentration of a protein solution required as medicine, or not (S3). In S3, the CPU 31 specifies a concentration at which the peak shape changes, reads out the specific concentration value from the storage unit 34 to the RAM 32, and compares the specified concentration with the concentration value. In a case where the concentration at which the peak shape changes is equal to or lower than the specific concentration value (S3: YES), the CPU 31 judges that the protein solution to be analyzed is not suitable as medicine (S4). In a case where a concentration at which the peak shape changes is equal to or lower than the specific concentration value, this indicates that the entire structure of protein is not maintained at a concentration required as medicine, and the protein solution is not suitable as medicine. Next, the CPU 31 causes the display unit 36 to display the judgment result (S5) and terminates the processing.

In a case where the peak shape at 1650 cm⁻¹ has not changed (S2: NO) or in a case where a concentration at which the peak shape changes exceeds the specific concentration value (S3: NO), the CPU 31 calculates an intensity ratio (856/837 cm⁻¹) of peaks at 856 cm⁻¹ and 837 cm⁻¹ included in Raman spectra of a plurality of concentrations (S6). Next, the CPU 31 specifies a concentration range in which the intensity ratio of 856/837 cm⁻¹ is substantially constant against change in concentration (S7). In S7, the CPU 31 calculates a variation of the intensity ratio of 856/837 cm⁻¹ against change in concentration and specifies a concentration range, in which the variation falls in a predetermined fine range that is preset, as a concentration range in which the intensity ratio is substantially constant. Within this concentration range, it is clear that interaction between protein molecules in a protein solution is substantially saturated, and protein may possibly have been denatured.

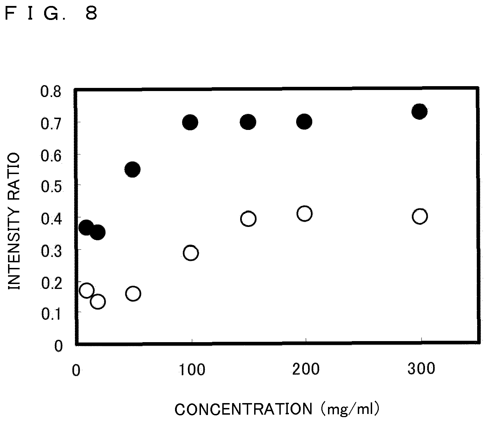

FIG. 8 is a characteristic diagram illustrating an example (black circles) of change in intensity ratio (856/837 cm⁻¹) of peaks at 856 cm⁻¹ and 837 cm⁻¹ included in the Raman band of tyrosine against concentration, and an example (white circles) of change in intensity ratio (870/877 cm⁻¹) of peaks at 870 cm⁻¹ and 877 cm⁻¹ included in the Raman band of tryptophan against concentration. The horizontal axis represents concentration, while the vertical axis represents intensity ratio of peaks. FIG. 8 illustrates the intensity ratio of peaks calculated from Raman spectra of lysozyme solutions. In the figure, the intensity ratio of 856/837 cm⁻¹ is represented by black circles, while the intensity ratio of 870/877 cm⁻¹ is represented by white circles. In the example illustrated in FIG. 8, the intensity ratio of 856/837 cm⁻¹ is substantially constant within a concentration range equal to or higher than 100 mg/ml.

Next, the CPU 31 specifies a peak width at 1555 cm⁻¹ included in Raman spectra of a plurality of concentrations (S8) and specifies a concentration range in which the peak width is substantially constant against change in concentration (S9). In S9, the CPU 31 calculates a variation of the peak width at 1555 cm⁻¹ against change in concentration and specifies a concentration range, in which the variation falls in a predetermined fine range that is preset, as a concentration range in which the peak width is substantially constant. The process of S6, S8, and S10 corresponds to a calculation unit in the present invention, and the process of S7, S9, and S11 corresponds to a first specification unit in the present invention.

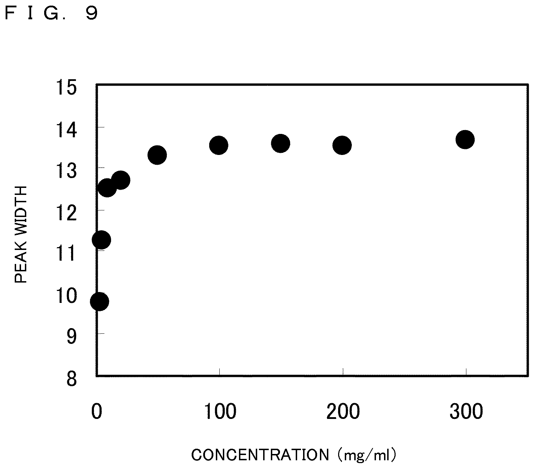

FIG. 9 is a characteristic diagram illustrating an example of change in peak width at 1555 cm⁻¹ included in the Raman band of tryptophan against concentration. The horizontal axis represents concentration, while the vertical axis represents peak width. FIG. 9 illustrates peak widths calculated from Raman spectra of lysozyme solutions. In the example illustrated in FIG. 9, the peak width at 1555 cm⁻¹ is substantially constant within a concentration range equal to or higher than 100 mg/ml. Within this concentration range, it is clear that interaction between protein molecules in a protein solution is substantially saturated, and protein may possibly have been denatured.

Next, the CPU 31 calculates an intensity ratio (870/877 cm⁻¹) of peaks at 870 cm⁻¹ and 877 cm⁻¹ included in Raman spectra of a plurality of concentrations (S10) and specifies a concentration range in which the intensity ratio of 870/877 cm⁻¹ is substantially constant against change in concentration (S11). In S11, the CPU 31 calculates a variation of intensity ratio of 870/877 cm⁻¹ against change in concentration and specifies a concentration range, in which the variation falls in a predetermined fine range that is preset, as a concentration range in which intensity ratio is substantially constant. In the example illustrated in FIG. 8, the intensity ratio of 870/877 cm⁻¹ is substantially constant within a concentration range equal to or higher than 150 mg/ml. Within this concentration range, it is clear that interaction between protein molecules in a protein solution is substantially saturated, and protein may possibly have been denatured.

Next, the CPU 31 specifies a threshold of concentration at which protein included in the protein solution to be analyzed can be denatured (S12). For example, the CPU 31 specifies the minimum value of concentration, which is included in at least one of concentration ranges specified in S7, S9, and S11, among a plurality of concentrations, for which Raman spectra have been acquired, as a threshold. In the example of a lysozyme solution, the threshold is 100 mg/ml. A concentration equal to or higher than the specified threshold is included in a concentration range specified in S7, S9, or S11. Therefore, at a concentration equal to or higher than the threshold, there is a high possibility that interaction between protein molecules in the protein solution is substantially saturated and protein has been denatured.

It is to be noted that the CPU 31 may perform processing for specifying a value, which is smaller than the minimum value of concentration included in at least one of concentration ranges specified in S7, S9, and S11, as the threshold. Although it is clear that protein is denatured within a concentration range which is specified in S7, S9, or S11 and in which an index that changes according to interaction between protein molecules is constant, there is a high possibility that denaturation of protein actually starts at a lower concentration. In order to exclude a protein solution in which protein is denatured at a high concentration more reliably, it is preferable to set a lower criterion for determining whether protein is denatured or not. Hence, in S12, a value smaller than the minimum value of a concentration range, in which the index is constant, is specified as the threshold based on this minimum value according to a predetermined rule. For example, the CPU 31 specifies a value, which is obtained by subtracting a predetermined positive value from the minimum value of concentration included in at least one of the concentration ranges specified in S7, S9, and S11, as the threshold. Moreover, for example, the CPU 31 may specify a value, which is obtained by multiplying the minimum value of concentration included in at least one of the concentration ranges specified in S7, S9, and S11 by a predetermined value that is larger than 0 and smaller than 1, as the threshold. The process of S12 corresponds to a second specification unit in the present invention.

Next, the CPU 31 compares the specified concentration value with a specific concentration value, which indicates a concentration of the protein solution required as medicine (S13). In S13, the CPU 31 compares the concentration value, which is read out from the storage unit 34, with the threshold. The process of S13 corresponds to a comparison unit in the present invention. The CPU 31 causes the display unit 36 to display the comparison result (S14) and terminates the processing. As described above, an analyzer determines whether the protein solution to be analyzed is suitable as medicine or not on the basis of the comparison result displayed at the display unit 36. For example, in a case where the specific concentration value is equal to or larger than the threshold, the analyzer judges that the protein solution to be analyzed is not suitable as medicine, since protein is denatured at a concentration required as medicine and an effect as medicine changes. It is to be noted that the analysis device 3 may execute the process of S6 to S7, the process of S8 to S9, and the process of S10 to S11 among the processes of S1 to S14 in another order or in parallel. Moreover, the analysis device 3 may execute processing, which is obtained by omitting any one or two of the process of S6 to S7, the process of S8 to S9, and the process of S10 to S11.

As described above in detail, in this embodiment, Raman spectra of protein solutions having a plurality of concentrations are acquired, and a threshold of concentration at which protein in the protein solution can be denatured is specified on the basis of the Raman spectra and is compared with a concentration of the protein solution required as medicine. By using an intensity ratio of 856/837 cm⁻¹, a peak width at 1555 cm⁻¹, or an intensity ratio of 870/877 cm⁻¹ as an index indicating the ratio of protein molecules interacting with other protein molecules, it is possible to specify a threshold of concentration at which interaction between protein molecules is saturated and protein can be denatured. By comparing the specified threshold with a concentration of the protein solution required as medicine, it is possible to evaluate whether the protein solution is suitable for medicine or not by, for example, judging that the protein solution is not suitable as medicine in a case where a concentration required as medicine is equal to or higher than the threshold. Accordingly, this embodiment makes it possible to easily evaluate whether a protein solution containing protein such as an antibody dissolved therein is suitable for medicine or not.

In the present invention, it is to be noted that the reciprocal of the intensity ratio of 870/877 cm⁻¹ or the intensity ratio of 856/837 cm⁻¹ may be utilized as an index. Moreover, although a form wherein peaks at 856 cm⁻¹, 837 cm⁻¹, 870 cm⁻¹, 877 cm⁻¹, 1555 cm⁻¹, and 1650 cm⁻¹ in Raman spectra are utilized has been described as this embodiment, the value of Raman shift at these peaks may possibly shift depending on conditions of the protein solution. Even when the value of Raman shift at these peaks shifts, it is possible to similarly execute an analysis method of a protein solution. Moreover, although a form wherein the control unit 24 and the analysis device 3 are separated from each other has been described as this embodiment, the Raman spectroscopic device may have a form wherein the control unit 24 and the analysis device 3 are united with each other. Moreover, although a form wherein the analysis device 3 is included in the Raman spectroscopic device has been described as this embodiment, the analysis device 3 may have a form separated from the Raman spectroscopic device. An analysis device 3 according to this form receives and stores Raman spectrum data generated at the Raman spectroscopic device. Moreover, it is also possible with the present invention to analyze a protein solution without using an analysis device 3.

### (Embodiment 2)

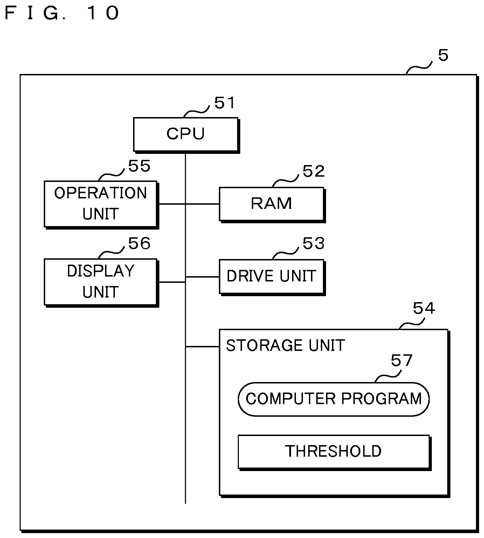

In this embodiment, an analyzer evaluates whether a protein solution is suitable for medicine or not in a case where he wants to use the protein solution having a specific concentration as medicine. FIG. 10 is a block diagram illustrating the inner structure of an analysis device 5 according to Embodiment 2. The analysis device 5 is provided with a CPU 51, a RAM 52, a drive unit 53, a storage unit 54, an operation unit 55, and a display unit 56. The CPU 51 causes the drive unit 53 to read a computer program 57 recorded in a recording medium, which is not illustrated in the figure, and causes the storage unit 54 to store the read computer program 57. The computer program 57 may be downloaded from outside of the analysis device 5. The CPU 51 loads the computer program 57 from the storage unit 54 to the RAM 52 as needed and executes processing necessary for the analysis device 5 according to the loaded computer program 57. By an analysis method similar to Embodiment 1, a threshold of concentration at which protein can be denatured is preliminarily specified regarding a protein solution, which is a candidate to be used as medicine. The specified threshold is preliminarily stored in the storage unit 54. For example, a threshold is stored in association with each type of a plurality of proteins.

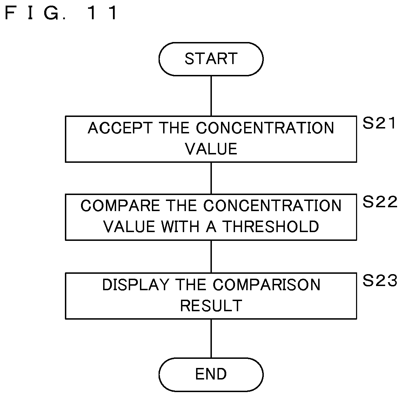

FIG. 11 is a flowchart illustrating procedures of processing to be executed by the analysis device 5 according to Embodiment 2. The CPU 51 executes the following processing according to the computer program 57. The concentration of a protein solution to be analyzed is preliminarily measured. An analyzer operates the operation unit 55 to input a concentration value of the protein solution. The CPU 51 accepts the concentration value at the operation unit 55 and causes the RAM 52 to store the accepted concentration value (S21). At this time, the analysis device 5 may accept the type of protein together with the concentration value and may further accept conditions of the protein solution. Next, the CPU 51 compares the accepted concentration value with a threshold of concentration at which protein can be denatured (S22). In S22, the CPU 51 reads out a threshold from the storage unit 54 and compares the read-out threshold with the concentration value. For example, the CPU 51 reads out a threshold which is stored in the storage unit 54 in association with the accepted type of protein. The process of S22 corresponds to a comparison unit in the present invention. The CPU 51 causes the display unit 56 to display the comparison result (S23) and terminates the processing. The analyzer determines whether the protein solution to be analyzed is suitable as medicine or not in a manner similar to Embodiment 1 on the basis of the comparison result displayed at the display unit 56. As described above, it is also possible with this embodiment to easily evaluate whether a protein solution containing protein such as an antibody dissolved therein is suitable for medicine or not.

### (Embodiment 3)

Illustrated in this embodiment is a form wherein a protein solution to be analyzed is an IgG (Immunoglobulin G) solution. In this embodiment, an example in which an aqueous solution of IgG is to be analyzed is illustrated mainly. IgG is a type of an antibody and is sometimes utilized for antibody drug. An analysis method of an IgG solution is similar to the case of Embodiment 1 illustrated in FIG. 1. The structure of a Raman spectroscopic device and an analysis device 3 to be used for analysis of an IgG solution is similar to the case of Embodiment 1 illustrated in FIGS. 2 and 4. An analyzer prepares IgG solutions having a plurality of concentrations and acquires Raman spectra of the IgG solutions using the Raman spectroscopic device. The Raman spectroscopic device generates a Raman spectrum for the IgG solution having each concentration. The analysis device 3 stores Raman spectrum data representing a Raman spectrum of each concentration in the storage unit 34. The analyzer inputs concentration data, which indicates the concentration of the IgG solutions, and the CPU 31 causes the storage unit 34 to store the concentration data. Raman spectrum data, which represents a Raman spectrum of each concentration, and each concentration indicated by the concentration data are associated with each other. A Raman spectrum and a concentration are associated with each other when an analyzer operates the operation unit 35. Moreover, the storage unit 34 stores a specific concentration value, which indicates a concentration of an IgG solution required as medicine.

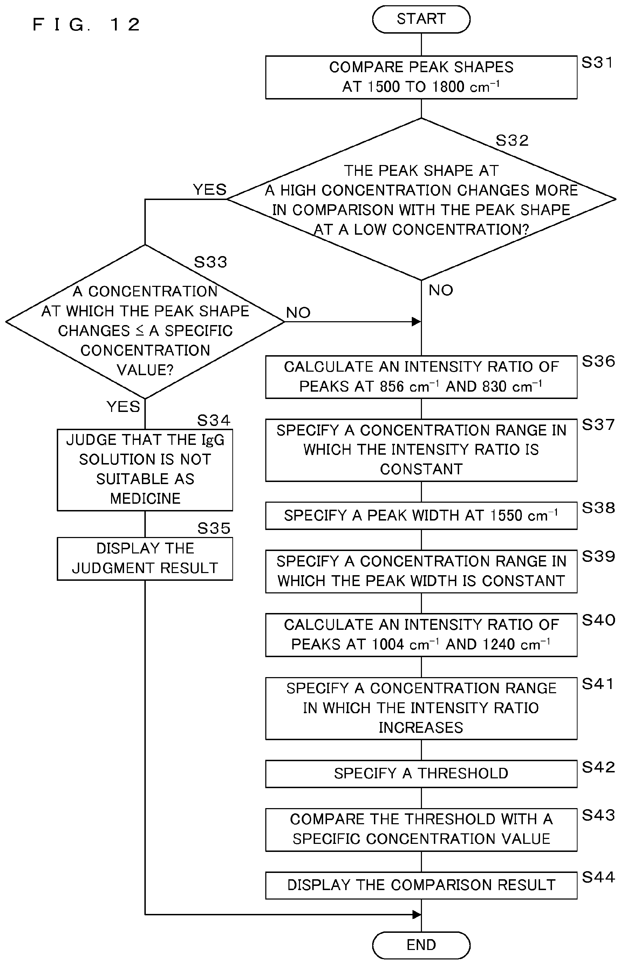

The analysis device 3 performs processing for analyzing an IgG solution on the basis of change of a predetermined peak included in Raman spectra against increase in concentration. FIG. 12 is a flowchart illustrating procedures of processing to be executed by the analysis device 3 according to Embodiment 3. The CPU 31 of the analysis device 3 executes the following processing according to the computer program 40. The CPU 31 reads out Raman spectrum data of a plurality of concentrations from the storage unit 34 to the RAM 32 and compares peak shapes at 1500 to 1800 cm⁻¹ included in Raman spectra of a plurality of concentrations (S31).

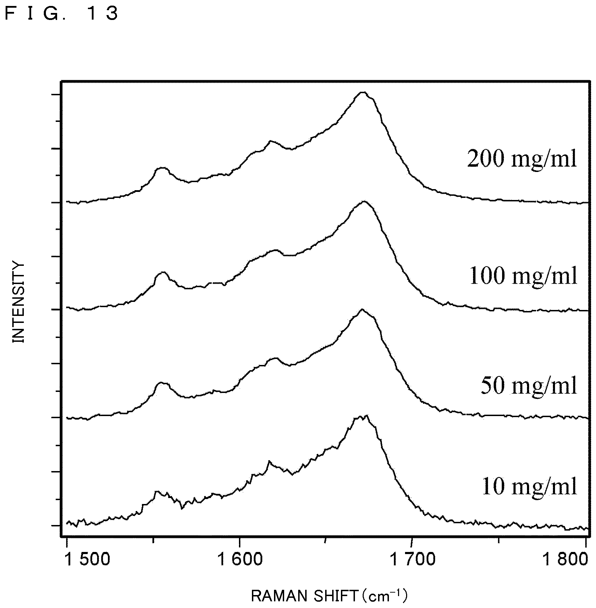

FIG. 13 is a characteristic diagram illustrating an example of Raman spectra of amide I of aqueous solutions of IgG. The horizontal axis in the figure represents Raman shift using a unit of wavenumber (cm⁻¹), while the vertical axis represents normalized intensity. FIG. 13 illustrates bands at 1500 to 1800 cm⁻¹ included in Raman spectra of aqueous solutions of IgG having the respective concentrations of 10, 50, 100, and 200 (mg/ml). Raman spectra of the respective concentrations are shown with baselines thereof being different from each other. Peaks which appear in bands at 1500 to 1800 cm⁻¹ included in the Raman spectra of IgG solutions are peaks of amide I bands resulting from an amide group in IgG. Peaks of this band appears when the entire structure of IgG is maintained.

Next, the CPU 31 determines whether the spectrum shape at a high concentration changes more in comparison with the spectrum shape at a low concentration or not on the basis of the comparison result (S32). For example, the CPU 31 calculates a difference square sum between normalized Raman spectra of a plurality of concentrations in S31, and compares the difference square sum with a predetermined allowable value in S32. The CPU 31 judges that the spectrum shape has changed in a case where the difference square sum exceeds the allowable value. In a case where the peak shape at 1500 to 1800 cm⁻¹ changes more at a high concentration in comparison with a low concentration, this indicates that the entire structure of IgG is not maintained in an IgG solution having a high concentration. In the example of an aqueous solution of IgG illustrated in FIG. 13, no change was observed in the peak shape at 1500 to 1800 cm⁻¹ in any of concentrations of 10, 50, 100, and 200 (mg/ml). That is, in the example illustrated in FIG. 13, the entire structure of IgG is maintained in an aqueous solution of IgG at any concentration.

In a case where the peak shape at 1500 to 1800 cm⁻¹ has changed (S32: YES), the CPU 31 determines whether a concentration at which the peak shape at 1500 to 1800 cm⁻¹ changes more in comparison with a case of a low concentration is equal to or smaller than a specific concentration value, which indicates a concentration of an IgG solution required as medicine, or not (S33). In S33, the CPU 31 specifies a concentration at which the spectrum shape changes, reads out a specific concentration value from the storage unit 34 to the RAM 32, and compares the specified concentration with the concentration value. In a case where the concentration at which the spectrum shape changes is equal to or lower than the specific concentration value (S33: YES), the CPU 31 judges that the IgG solution to be analyzed is not suitable as medicine (S34). In such a case, it is assumed that the entire structure of IgG is not maintained at a concentration required as medicine, and the IgG solution is not suitable as medicine. Next, the CPU 31 causes the display unit 36 to display the judgement result (S35) and terminates the processing.

In a case where the peak shape at 1500 to 1800 cm⁻¹ has not changed (S32: NO) or in a case where a concentration at which the spectrum shape changes exceeds the specific concentration value (S33: NO), the CPU 31 calculates an intensity ratio (856/830 cm⁻¹) of peaks at 856 cm⁻¹ and 830 cm⁻¹ included in Raman spectra of a plurality of concentrations (S36).

The peaks at 856 cm⁻¹ and 830 cm⁻¹ included in Raman spectra of IgG solutions result from tyrosine. The peak positions are slightly different from the case of lysozyme illustrated in Embodiment 1. An intensity ratio (856/830 cm⁻¹) obtained by dividing the peak intensity at 856 cm⁻¹ by the peak intensity at 830 cm⁻¹ indicates the degree to which an OH group of tyrosine in IgG acts as a hydrogen acceptor while acting also as a hydrogen donner. In a case where the concentration of an IgG solution is relatively low, an OH group of tyrosine acts as a hydrogen donner, and the intensity ratio of 856/830 cm⁻¹ is relatively small. As the concentration of the IgG solution becomes high, IgG molecules interact with each other, the OH group of tyrosine starts acting as an acceptor, and the intensity ratio of 856/830 cm⁻¹ becomes large. That is, the intensity ratio of 856/830 cm⁻¹ indicates the ratio of IgG molecules interacting with other IgG molecules. At a high concentration above a certain degree, interaction between IgG molecules is saturated, and the intensity ratio of 856/830 cm⁻¹ is substantially constant.

Next, the CPU 31 specifies a concentration range in which the intensity ratio of 856/830 cm⁻¹ is substantially constant against change in concentration (S37). In S37, the CPU 31 calculates a variation of intensity ratio of 856/830 cm⁻¹ against change in concentration and specifies a concentration range, in which the variation falls in a predetermined fine range that is preset, as a concentration range in which the intensity ratio is substantially constant. Within this concentration range, it is clear that interaction between IgG molecules is substantially saturated, and IgG may possibly have been denatured.

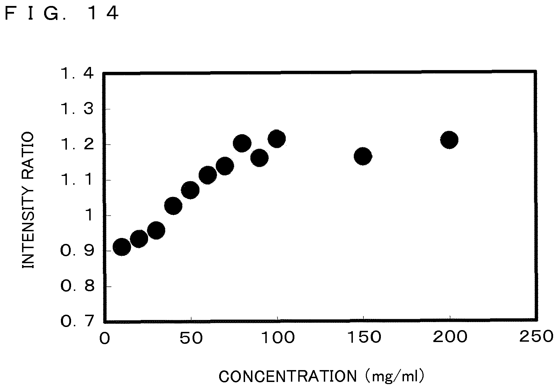

FIG. 14 is a characteristic diagram illustrating an example of change in intensity ratio of 856/830 cm⁻¹ obtained from Raman spectra of tyrosine in aqueous solutions of IgG against concentration. The horizontal axis represents concentration, while the vertical axis represents intensity ratio of a peak. In the example illustrated in FIG. 14, the intensity ratio of 856/830 cm⁻¹ is substantially constant within a concentration range equal to or higher than 100 mg/ml.

Next, the CPU 31 specifies a peak width at 1550 cm⁻¹ included in Raman spectra of a plurality of concentrations (S38). A peak at 1550 cm⁻¹ included in each Raman spectrum of an IgG solution results from C=C stretching vibration of an indole ring in tryptophan. The peak position is slightly different from the case of lysozyme illustrated in Embodiment 1. The peak width at 1550 cm⁻¹ indicates a distribution width of a torsion angle between C₂=C₃ and C_{α}-C_{β} illustrated in FIG. 6. As the concentration of an IgG solution becomes high, interaction between IgG molecules causes change in the torsion angle, increase in dispersion of the torsion angle, and widening of the peak width at 1550 cm⁻¹. That is, the peak width at 1550 cm⁻¹ also indicates the ratio of IgG molecules interacting with other IgG molecules. At a high concentration above a certain degree, interaction between IgG molecules is saturated, and the peak width is substantially constant.

Next, the CPU 31 specifies a concentration range in which the peak width at 1550 cm⁻¹ is substantially constant against change in concentration (S39). In S39, the CPU 31 calculates a variation of peak width at 1550 cm⁻¹ against change in concentration and specifies a concentration range, in which a the variation falls in a predetermined fine range that is preset, as a concentration range in which a peak width is substantially constant.

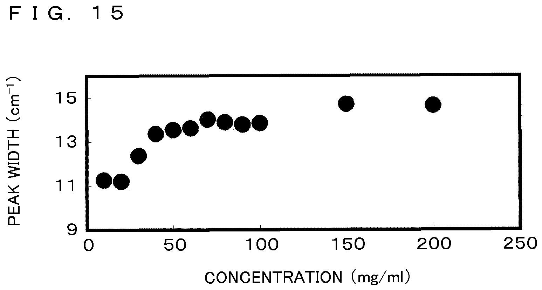

FIG. 15 is a characteristic diagram illustrating an example of change in peak width at 1550 cm⁻¹ obtained from Raman spectra of tryptophan in aqueous solutions of IgG against concentration. The horizontal axis represents concentration, while the vertical axis represents peak width using a unit of wavenumber (cm⁻¹). In the example illustrated in FIG. 15, the peak width at 1550 cm⁻¹ is substantially constant within a concentration range equal to or higher than 100 mg/ml. Within a concentration range in which the peak width at 1550 cm⁻¹ is substantially constant, it is clear that interaction between IgG molecules in an IgG solution is substantially saturated, and IgG may possibly have been denatured.

Next, the CPU 31 calculates an intensity ratio (1004/1240 cm⁻¹) of peaks at 1004 cm⁻¹ and 1240 cm⁻¹ included in Raman spectra of a plurality of concentrations (S40). A peak at 1004 cm⁻¹ included in each Raman spectrum of an IgG solution results from phenylalanine. Phenylalanine is one of amino acids, which constitute IgG. Moreover, a peak at 1240 cm⁻¹ is a peak of amid III resulting from an amide group in IgG. The peak intensity at 1004 cm⁻¹ changes when the environment surrounding phenylalanine changes. Specifically, when IgG aggregates irreversibly, interaction with phenylalanine becomes strong, and the peak intensity at 1004 cm⁻¹ increases. The peak intensity at 1240 cm⁻¹ is used as the standard for the peak intensity at 1004 cm⁻¹. An intensity ratio (1004/1240 cm⁻¹) obtained by dividing the peak intensity at 1004 cm⁻¹ by the peak intensity at 1240 cm⁻¹ indicates the intensity of interaction with phenylalanine in IgG. In a case where the concentration of an IgG solution is relatively low, interaction with phenylalanine is weak, and the intensity ratio of 1004/1240 cm⁻¹ is substantially constant. As the concentration of an IgG solution becomes high, IgG aggregates irreversibly, interaction with phenylalanine becomes strong, and the intensity ratio of 1004/1240 cm⁻¹ increases according to concentration. That is, the intensity ratio of 1004/1240 cm⁻¹ indicates the degree of irreversible aggregation of IgG.

Next, the CPU 31 specifies a concentration range in which the intensity ratio of 1004/1240 cm⁻¹ increases according to increase in concentration (S41). In S41, the CPU 31 calculates a variation of intensity ratio at 1004/1240 cm⁻¹ against change in concentration and specifies a concentration range, in which the variation falls in a predetermined fine range that is preset, as a concentration range in which the intensity ratio increases according to increase in concentration.

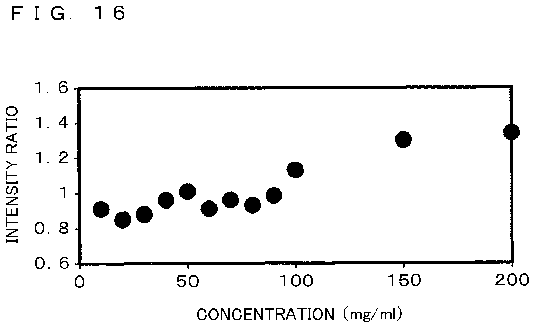

FIG. 16 is a characteristic diagram illustrating an example of change in intensity ratio of 1004/1240 cm⁻¹ obtained from Raman spectra of aqueous solutions of IgG against concentration. The horizontal axis represents concentration, while the vertical axis represents intensity ratio of a peak. In the example illustrated in FIG. 16, the intensity ratio of 1004/1240 cm⁻¹ is substantially constant within a concentration range equal to or lower than 90 mg/ml and increases according to concentration within a concentration range equal to or higher than 100 mg/ml. Within the concentration range in which the intensity ratio of 1004/1240 cm⁻¹ increases according to concentration, it is clear that IgG aggregates irreversibly in an IgG solution, and IgG may possibly have been denatured. By using the intensity ratio of 1004/1240 cm⁻¹ as an index which increases and decreases according to interaction between IgG molecules, it becomes possible to specify a threshold of concentration at which IgG can aggregate irreversibly and be denatured.

Next, the CPU 31 specifies a threshold of concentration at which IgG included in the IgG solution to be analyzed can be denatured (S42). For example, the CPU 31 specifies the minimum value of concentration included in at least one of concentration ranges, which are specified in S37, S39, and S41, among a plurality of concentrations, for which Raman spectra have been acquired, as a threshold. In the example of an aqueous solution of IgG, the threshold is 100 mg/ml. At a concentration equal to or higher than the threshold, there is a high possibility that interaction between IgG molecules in an IgG solution is substantially saturated and IgG has been denatured.

It is to be noted that the CPU 31 may perform processing for specifying a value, which is smaller than the minimum value of concentration included in at least one of concentration ranges specified in S37, S39, and S41, as the threshold. For example, the CPU 31 specifies a value, which is obtained by subtracting a predetermined positive value from the minimum value of concentration included in at least one of concentration ranges specified in S37, S39, and S41, as the threshold. Moreover, for example, the CPU 31 may specify a value, which is obtained by multiplying the minimum value of concentration included in at least one of concentration ranges specified in S37, S39, and S41 by a predetermined positive value that is smaller than 1, as the threshold. The process of S36, S38, and S40 corresponds to a calculation unit, and the process of S37, S39, S41, and S42 corresponds to a first specification unit in the present invention.

Next, the CPU 31 compares the specified concentration value with a specific concentration value, which indicates a concentration of an IgG solution required as medicine (S43). In S43, the CPU 31 compares a concentration value, which has been read out from the storage unit 34, with the threshold. The process of S43 corresponds to a comparison unit. The CPU 31 causes the display unit 36 to display the comparison result (S44) and terminates the processing. As described above, an analyzer determines whether the IgG solution to be analyzed is suitable as medicine or not on the basis of the comparison result displayed at the display unit 36. In a case where the specific concentration value is equal to or larger than the threshold, for example, the analyzer judges that the IgG solution to be analyzed is not suitable as medicine, since IgG is denatured at a concentration required as medicine and an effect as medicine changes. It is to be noted that the analysis device 3 may execute the process of S36 to S37, the process of S38 to S39, and the process of S40 to S41 among the processes of S31 to S44 in another order or in parallel. Moreover, the analysis device 3 may execute processing, which is obtained by omitting any one or two of the process of S36 to S37, the process of S38 to S39, and the process of S40 to S41.

Although a form wherein a concentration range in which the intensity ratio of 1004/1240 cm⁻¹ increases according to increase in concentration is specified in S41 is illustrated in the above processing, it is to be noted that the analysis device 3 may perform processing for specifying a concentration range, in which the intensity ratio of 1004/1240 cm⁻¹ is substantially constant against change in concentration, instead of S41. In this case, the analysis device 3 specifies the minimum value of the lower limits of concentration ranges, which are specified in S37 and S39, and of the upper limits of concentration ranges, which are specified in the processes to be performed instead of S41, as a threshold in S42. Moreover, a value smaller than the minimum value may be used as a threshold.

As described above in detail, in this embodiment, Raman spectra of IgG solutions having a plurality of concentrations are acquired, and a threshold of concentration at which IgG can be denatured is specified on the basis of the Raman spectra. By comparing the specified threshold with a concentration required as medicine, it is possible to evaluate whether a specific IgG solution is suitable for medicine or not by, for example, judging that the IgG solution is not suitable as medicine in a case where a concentration required as medicine is equal to or higher than the threshold. Accordingly, it becomes possible with this embodiment to easily evaluate whether a solution including an antibody such as IgG is suitable for medicine or not.

In an analysis method of a protein solution such as an IgG solution, it is to be noted that the reciprocal of the intensity ratio of 1004/1240 cm⁻¹ or the intensity ratio of 856/830 cm⁻¹ may be utilized as an index. Moreover, a peak at a wavenumber other than 1240 cm⁻¹ may be used as the standard of a peak at 1004 cm⁻¹.
Moreover, the Raman spectroscopic device may have a form wherein the control unit 24 and the analysis device 3 are united with each other. Moreover, the analysis device 3 may have a form separated from the Raman spectroscopic device. Moreover, in the analysis method, it is also possible to analyze an IgG solution without using an analysis device 3. Moreover, although an aqueous solution of IgG is to be analyzed in this embodiment, an IgG solution other than an aqueous solution, such as a solution obtained by dissolving IgG in a buffer solution, may be analyzed in the analysis method. Moreover, a solution of an antibody other than IgG may also be analyzed in the analysis method. Moreover, although a form wherein peaks at 856 cm⁻¹, 830 cm⁻¹, 1004 cm⁻¹, 1240 cm⁻¹, and 1550 cm⁻¹ in Raman spectra are utilized is illustrated in this embodiment, the value of Raman shift of these peaks sometimes shifts depending on conditions of an antibody solution or the type of an antibody. The analysis method can be similarly executed even when the value of Raman shift of these peaks shifts.

### [Description of Reference Numerals]

- 1: Protein Solution
- 21: Light Source
- 22: Spectroscope
- 23: Detector
- 24: Control Unit
- 3: Analysis Device
- 31, 51: CPU
- 32, 52: RAM
- 34, 54: Storage Unit
- 35, 55: Operation Unit
- 36, 56: Display Unit
- 4: Recording Medium
- 40, 57: Computer Program

## Claims

1. An analysis method for analyzing a protein solution utilizing Raman spectroscopy,
**characterized by** comprising the steps of:
acquiring a Raman spectrum of each of protein solutions, which contain a specific protein dissolved therein and have a plurality of concentrations, by Raman spectroscopy;
calculating an index, which increases and decreases according to interaction between protein molecules in the protein solution, based on one or a plurality of predetermined peaks included in the Raman spectrum for each concentration;
specifying a concentration range, in which a variation of the index against increase in concentration falls in a predetermined range, based on the index calculated regarding each concentration;
specifying a threshold of concentration, which is to be used as a criterion for determining whether the specific protein is denatured or not, based on the concentration range; and
comparing the threshold with a specific concentration value set for the specific protein.

2. An analysis method for analyzing a protein solution utilizing Raman spectroscopy,
**characterized by** comprising the steps of:
acquiring a Raman spectrum of each of protein solutions, which contain a specific protein dissolved therein and have a plurality of concentrations, by Raman spectroscopy;
calculating an index, which increases and decreases according to interaction between protein molecules in the protein solution, based on one or a plurality of predetermined peaks included in the Raman spectrum for each concentration; and
specifying a threshold of concentration, which is to be used as a criterion for determining whether the specific protein is denatured or not, based on change in the index against change in concentration.

3. The analysis method according to Claim 1 or 2, wherein the index is an intensity ratio of two predetermined peaks in the Raman spectrum, the intensity ratio indicating a degree to which an OH group of tyrosine in protein acts as a hydrogen acceptor while acting also as a hydrogen donner.

4. The analysis method according to any one of Claims 1 to 3, wherein
the index is an intensity ratio of two predetermined peaks in the Raman spectrum, the intensity ratio indicating a degree to which an N-H site of an indole ring included in tryptophan in protein acts as a hydrogen acceptor while acting also as a hydrogen donner.

5. The analysis method according to any one of Claims 1 to 4, wherein
the index is a width of a predetermined peak in the Raman spectrum, the width indicating a width of a predetermined structural angle of tryptophan in protein.

6. The analysis method according to any one of Claims 1 to 5, wherein
the index is an intensity ratio of a predetermined peak resulting from phenylalanine in protein and a predetermined peak resulting from an amide group.

7. The analysis method according to any one of Claims 1 to 6, further comprising the steps of:
comparing a peak shape of a predetermined peak resulting from an amide group in protein between Raman spectra regarding a plurality of concentrations;
determining whether the peak shape at a high concentration changes more in comparison with the peak shape at a low concentration or not; and
judging that the protein solution is not suitable for medicine at a concentration equal to or higher than a concentration at which the peak shape changes in a case where the peak shape at the high concentration changes more in comparison with the peak shape at the low concentration.

8. The analysis method according to any one of Claims 1 to 7, further comprising the steps of:
determining whether the protein solution is suitable for medicine or not based on a result of comparison between the threshold and the specific concentration value; and
repeating analysis of the protein solution, which is prepared by adjusting a component other than the specific protein, in a case where the protein solution is not suitable for medicine.

9. An analysis device for analyzing a protein solution based on a Raman spectrum,
**characterized by** comprising:
a storage unit configured to store a specific concentration value set for a specific protein, and a Raman spectrum acquired for each of protein solutions, which contain the specific protein dissolved therein and have a plurality of concentrations;
a calculation unit configured to calculate an index, which increases and decreases according to interaction between protein molecules in the protein solution, based on one or a plurality of predetermined peaks included in the Raman spectrum for each concentration;
a first specification unit configured to specify a concentration range, in which a variation of the index against increase in concentration falls in a predetermined range, based on the index calculated regarding each concentration;
a second specification unit configured to specify a threshold of concentration, which is to be used as a criterion for determining whether the specific protein is denatured or not, based on the concentration range; and
a comparison unit configured to compare the threshold with the specific concentration value.

10. An analysis device for analyzing a protein solution based on a Raman spectrum,
**characterized by** comprising:
a storage unit configured to store a specific concentration value set for a specific protein, and a Raman spectrum acquired for each of protein solutions, which contain the specific protein dissolved therein and have a plurality of concentrations;
a calculation unit configured to calculate an index, which increases and decreases according to interaction between protein molecules in the protein solution, based on one or a plurality of predetermined peaks included in the Raman spectrum for each concentration; and
a specification unit configured to specify a threshold of concentration, which is to be used as a criterion for determining whether the specific protein is denatured or not, based on change in the index against change in concentration.

11. An analysis device for analyzing a protein solution, **characterized by** comprising:
a storage unit configured to store a threshold of concentration, which is preliminarily specified utilizing Raman spectroscopy for a protein solution containing a specific protein dissolved therein and is to be used as a criterion for determining whether the specific protein is denatured or not; and
a comparison unit configured to compare a specific concentration value, which is set for the specific protein, with the threshold.
